# EUROPÄISCHE PATENTANMELDUNG

(11) **EP 4 018 959 A1**
(43) Veröffentlichungstag der Anmeldung: **29.06.2022**
(21) Anmeldenummer: 21213392.0
(22) Anmeldetag: 09.12.2021
(51) Int. Cl.: A61B 34/37, A61B 34/00

(54) **ROBOTISCHES OPERATIONSSYSTEM**

(30) Priorität: 22.12.2020 DE 102020134626
(71) Anmelder: avateramedical GmbH, 07745 Jena (DE)
(72) Erfinder: Gaßner, Tobias, 76709 Kronau (DE); Tauro, Ricardo A., 76187 Karlsruhe (DE); Luksch, Tobias, 76761 Rülzheim (DE); Spittel, Lars, 98693 Ilmenau (DE)
(74) Vertreter: Gleim Petri Oehmke Patent- und Rechtsanwaltspartnerschaft mbB

(57) **Zusammenfassung**

Die Erfindung betrifft ein Verfahren zur Steuerung mindestens eines Manipulatorarms (2) eines robotischen Operationssystems und eines von dem mindestens einen Manipulatorarm (2) gehaltenen Instruments (7) mittels einer Eingabeeinheit (16). Die Eingabeeinheit (16) weist eine mehrgelenkige, kinematische Kette mit aktiven und/oder passiven Gelenken (17, 18, 19, 20, 21) sowie ein offenes Ende auf. Das offene Ende ist als Handstück (22) mit mindestens einem ersten passiven Gelenk (23) ausgebildet. Über die Bewegung des Handstück (22) steuert ein Operateur Bewegungen des Instruments (7). Dazu ist das erste passive Gelenke (23) mit einem ersten Lagesensor ausgestaltet, wobei Positionssignale des ersten Lagesensors zur Steuerung der Bewegung des Instruments (7) verwendet werden. Aus einer zeitlichen Abfolge der Positionssignale des ersten Lagesensors wird ein erster Messwert berechnet und mit mindestens einem vorgegebenen ersten Schwellwert verglichen. Die Steuerung der Bewegung des Instruments (7) wird unterbrochen, falls der erste Messwert kleiner als der erste Schwellwert ist. Die Erfindung betrifft außerdem ein robotisches Operationssystem, welches dieses Verfahren verwendet.

## Beschreibung

### Gebiet der Erfindung

Die Erfindung betrifft ein robotisches Operationssystem mit mindestens einem Manipulatorarm sowie ein Verfahren zur Steuerung des mindestens einen Manipulatorarms und eines von dem mindestens einen Manipulatorarm gehaltenen Instruments mittels einer Eingabeeinheit, und betrifft das Problem einer möglichst genauen Erkennung, ob ein Operateur - im Betrieb in der Regel ein Chirurg - das Operationssystem bedient, die Bedienung vorbereitet oder von der Bedienung des Operationssystems ablässt. Eine möglichst genaue Erkennung hilft dabei, die Sicherheit des Systems gegen Bedienungsfehler, welche im schlimmsten Fall das Leben eines behandelten Patienten gefährden können, zu erhöhen.

Robotische Operationssysteme umfassen in der Regel eine Konsole zur Bedienung des Manipulatorarms. Die Konsole - auch als Kontrolleinheit bezeichnet - ist von dem übrigen Teil des Operationssystems mit einem oder mehreren Manipulatorarmen in der Regel räumlich getrennt, bzw. relativ zu diesem beweglich. Der Manipulatorarm ist als kinematische Kette mit einer Vielzahl von Gelenken ausgebildet, am Ende der Kette befindet sich ein Instrument in einer Instrumentenhalterung. Ein Teil der Gelenke des Manipulatorarms dient der Vorpositionierung und wird vor der Operation fixiert, während der übrige Teil bei der Operation mittels der Eingabeeinheit bewegt werden kann. Dieser Teil der Gelenke einschließlich der Instrumentenhalterung sowie ggf. einschließlich dem Instrument wird in der Konsole als eine Eingabeeinheit mit einer mehrgelenkigen kinematischen Kette mit aktiven und/oder passiven Gelenken abgebildet. Die Eingabeeinheit umfasst außerdem ein offenes Ende, welches sich an die mehrgelenkige kinematische Kette anschließt. Unter einem aktiven Gelenk versteht man dabei ein Gelenk, welches motorisch angetrieben ist. Bei einem aktiven Drehgelenk beispielsweise erfolgt die Drehung motorisch angetrieben, wobei der Motor in der Regel durch eine Steuereinheit angesteuert wird. Passive Gelenke hingegen sind nicht motorisch angetrieben, sondern sind frei beweglich, wobei die Bewegung entweder von Hand ausgelöst wird oder im Verbund mit anderen, aktiven Gelenken, erfolgt.

Die Bedienung der Eingabeeinheit ist dabei so eingestellt, dass sie möglichst leichtgängig bewegt werden kann; dies wird durch eine regelungstechnische Gewichtskraftkompensation erreicht, welche die alleinige Bewegungsunterstützung in der Eingabeeinheit ist, wenn die Eingabeeinheit mit einem Manipulatorarm gekoppelt ist. Üblicherweise verfügt eine Konsole über zwei Eingabeeinheiten, so dass ein Operateur zwei Manipulatorarme gleichzeitig bedienen kann, wie es auch bei einem am Operationstisch stehenden Chirurgen der Fall ist. Die Bewegungen des Instruments steuert der Operateur mittels eines Handstücks. Dabei muss ein Mechanismus vorgesehen werden, der sicherstellt, dass das Instrument auch nur dann bewegt wird, wenn es vom Operateur mittels der Eingabeeinheit gesteuert wird. Befindet sich der Operateur nicht an der Eingabeeinheit, so muss verhindert werden, dass unbeabsichtigte Bewegungen auf das Instrument übertragen werden. Somit muss detektiert werden, ob der Operateur bereit ist, dass Instrument mittels der Eingabeeinheit zu bedienen, oder nicht. Der Stand der Technik offenbart dazu bereits verschiedene Lösungsansätze.

### Stand der Technik

So wird in der US 2011/118748 A1 ein System offenbart, welches die Gegenwart einer Hand in der Nähe des Handstücks automatisch detektiert. Als Mittel werden beispielsweise kapazitive Schalter, Druckschalter oder Schalter, welche auf Infrarotbasis arbeiten, vorgeschlagen. In der WO 2018/162921 A1 befindet sich am Eingabegerät ein Schalter, mit welchem der Operateur die aktive Steuerung ein- oder ausschalten kann.

Auch in der US 2015/0100066 A1 werden Sensoren genannt, welche die Gegenwart einer Hand am Handstück überprüfen. Beispielhaft wird hier ein Hebel-Knopf-Mechanismus genannt. Die Steuerung der Bewegung des Instruments wird nur dann zugelassen, wenn die auf das Handstück ausgeübte Kraft einen definierten Mindestwert überschreitet, wozu ein weiterer Sensor notwendig ist, welcher die ausgeübte Kraft erkennt.

Bei dem in der US 2018/0168758 A1 beschriebenen Handstück sind eine Vielzahl von Abstandssensoren verbaut, um zu erkennen, ob die Hand des Operateurs das Handstück greift. Für Beispiele solcher Sensoren wird allgemein auf optische oder kapazitive Sensoren verwiesen. Ein ähnlicher Ansatz wird auch in der WO 2019/217882 A1 verfolgt, wobei hier optische Sensoren oder Drucksensoren als Beispiel genannt werden.

Ein anderer Ansatz wird in der US 2003/0060927 A1 verfolgt. Hier werden die angetriebenen Gelenke dazu verwendet festzustellen, ob ein Operateur das Handstück berührt oder nicht. Dazu werden die aktiven Gelenke durch den Motor in Schwingungen mit sehr niedrigen Amplituden versetzt. Greift ein Operateur das Handstück, so ändert sich das Schwingungsmuster. Das Schwingungsmuster kann mittels eines Gelenkssensors, beispielsweise eines Potentiometers, detektiert werden.

Die im Stand der Technik beschriebenen Mechanismen sind gerade dann, wenn auf eine Leichtgängigkeit der Eingabeeinheit geachtet werden soll mitunter zu ungenau, benötigen mehrere spezielle Sensoren, welche für die Bewegung der Eingabeeinheit nicht zwingend notwendig sind, oder aktive Gelenke, um Vibrationen erzeugen zu können, die sich ggf. als unangenehm bemerkbar machen könnten.

### Beschreibung der Erfindung

Aufgabe der Erfindung ist es daher, ein Verfahren zur Steuerung mindestens eines Manipulatorarms eines robotischen Operationssystems zu entwickeln, dass zum Erhalt der Leichtgängigkeit ohne oder mit möglichst wenig zusätzlichen Elementen eine Erkennung ermöglicht, ob eine Hand an der Eingabeeinheit aufliegt oder nicht. Eine weitere Aufgabe der Erfindung ist die Entwicklung eines entsprechenden robotischen Operationssystems.

Diese Aufgabe wird durch ein Verfahren zur Steuerung mindestens eines Manipulatorarms eines robotischen Operationssystems und eines von dem mindestens einen Manipulatorarm gehaltenen Instruments gelöst, bei dem die Steuerung mit einer Eingabeeinheit erfolgt, welche eine mehrgelenkige, kinematische Kette mit aktiven und/oder passiven Gelenken sowie ein offenes Ende aufweist. Das offene Ende der Eingabeeinheit ist als Handstück mit mindestens einem ersten passiven Gelenk ausgebildet. Über die Bewegung des Handstück steuert ein Operateur Bewegungen des Instruments. Dabei ist das erste passive Gelenk mit einem ersten Lagesensor ausgestaltet und Positionssignale des ersten Lagesensors werden zur Steuerung der Bewegung des Instruments verwendet. Aus einer zeitlichen Abfolge der Positionssignale des ersten Lagesensors wird ein erster Messwert berechnet und mit mindestens einem vorgegebenen ersten Schwellwert verglichen. Falls der erste Messwert kleiner als der erste Schwellwert ist, wird die Steuerung der Bewegung des Instruments unterbrochen.

Der erste Lagesensor ist zur Steuerung der Bewegung des Instruments bereits vorhanden, so dass kein zusätzliches Element verbaut werden muss. Nur die Steuerung muss entsprechend angepasst werden, um die zeitliche Abfolge der Positionssignale des ersten Lagesensors analysieren zu können. Im einfachsten Fall wird aus der zeitlichen Abfolge der Positionssignale eine Positionsdifferenz berechnet, hierfür sind mindestens zwei zeitlich aufeinanderfolgende Positionssignale notwendig. Ist diese Differenz kleiner als ein vorgegebener Schwellwert, der ebenfalls einer Positionsdifferenz entspricht, so wird die Steuerung der Bewegung des Instruments unterbrochen, d. h. eine aktive Veränderung der Position des Instruments wird unterbunden. Für die Art der Analyse gibt es verschiedene Verfahren, welche im Stand der Technik im Prinzip bekannt sind. Beispielsweise kann die Genauigkeit der Analyse verfeinert werden, wenn nicht nur zwei zeitlich aufeinanderfolgende Positionssignale verwendet werden, sondern mehrere Positionssignale, und dies fortlaufend. In diesem Fall wird nicht die reine Differenz der Positionssignale betrachtet, sondern ihre Standardabweichungen, wobei weiter zurückliegende Positionssignale schwächer - beispielsweise exponentiell schwächer - gewichtet werden, so dass diese länger zurückliegenden Signale den Wert der Standardabweichung umso geringer beeinflussen, je weiter sie zurückliegen.

In einer besonders bevorzugten Ausgestaltung wird der erste Schwellwert so vorgegeben, dass ein Unterschreiten des ersten Schwellwerts durch den ersten Messwert das Vorliegen eines physiologischen Tremors und damit einer unwillkürlichen Bewegung ausschließt. Beim physiologischen Tremor handelt es sich um eine hochfrequente und unwillkürliche Muskelbewegung mit bei Raumtemperatur oder darüber sehr niedriger Amplitude, die jeder Muskelbewegung überlagert ist. Bei kurzen Bewegungen mit einer großen Positionsänderung ist der physiologische Tremor ebenfalls vorhanden, wird jedoch von der Bewegung überlagert und ist dadurch kaum wahrnehmbar. Nur bei besonders anspruchsvollen Bewegungen nahe dem Stillhalten wird der physiologische Tremor in der Regel wahrnehmbar, bei Kälte werden die Amplituden stärker. Für die Festlegung des ersten Schwellwerts wird jedoch die Raumtemperatur zugrunde gelegt. Wenn sich der erste Schwellwert daher an dem physiologischen Tremor bzw. den durch den physiologischen Tremor verursachten Amplituden/Auslenkungen bzw. Positionsänderungen orientiert, lässt sich mit besonders hoher Genauigkeit ermitteln, ob der Operateur seine Hand noch am Handstück hat. Liegt der erste Messwert daher unter dem ersten Schwellwert, so wird dies von der Steuerung als Loslassen des Handstücks interpretiert.

Anschließend werden vorteilhaft zumindest die aktiven Gelenke in ihren aktuellen Positionen fixiert, d. h. die Gelenke und Glieder der Eingabeeinheit lassen sich nicht mehr durch den Operateur bewegen, wenn dieser beispielsweis versehentlich und unbeabsichtigt versucht, eines der Glieder zu bewegen, ohne dabei das Handstück zu greifen. Die Fixierung kann bei den aktiven Gelenken über den motorischen Antrieb und eine entsprechende Bremse erfolgen. Auch die passiven Gelenke können durch entsprechende Feststell-Mechanismen fixiert werden, ansonsten sind die Teile der Gelenke im nicht fixierten Zustand jedoch frei gegeneinander beweglich. Die Fixierung der Gelenke erfolgt dabei bevorzugt unter Wirkung einer rückstellenden Kraft. Diese rückstellende Kraft wird bei den aktiven Gelenken mithilfe der Steuerung realisiert, die dafür sorgt, dass sich die Glieder, welche die Gelenke verbinden, wie Körper verhalten, die auf ihren jeweiligen Positionen federnd gehalten werden. Für den Fall, dass die Steuerung fälschlicherweise kurzzeitig ein Loslassen des Handstücks erkannt hat, wird der Operateur nicht beeinträchtigt, d. h. nach erkannter minimaler Positionsänderung wird die Steuerung der Gelenkpositionen durch den Operateur wieder aktiviert.

In einer besonders bevorzugten Ausführung dieser Variante wird die Steuerung der Bewegung des Instruments erst nach Ablauf einer vorgegebenen Zeitspanne unterbrochen. Ergibt die Messwertanalyse, dass der erste Messwert kleiner als der erste Schwellwert ist, so wird ein Zeitschalter gestartet, und nur wenn der erste Messwert innerhalb der vorgegebenen Zeitspanne nach Starten der Zeitschaltuhr permanent kleiner als der erste Schwellwert bleibt, wird die Steuerung nach Ablauf der vorgegebenen Zeitspanne - in der Regel nicht mehr als fünf Sekunden - unterbrochen und als Konsequenz die Kopplung zwischen der Eingabeeinheit und dem Manipulatorarm aufgehoben, so dass keine ungewollte Bewegung des Instruments oder der Instrumentenspitze mehr stattfinden kann.

In einer weiteren vorteilhaften Ausgestaltung des Verfahrens wird der erste Messwert zusätzlich mit einem zweiten Schwellwert verglichen, wobei der zweite Schwellwert so vorgegeben wird, dass ein Überschreiten des zweiten Schwellwerts durch den ersten Messwert einem Vorliegen einer bewusst vom Operateur initiierten Bewegung entspricht, wobei der zweite Schwellwert größer als der erste Schwellwert ist. Der zweite Schwellwert kann beispielsweise so festgelegt werden, dass er einer möglichen Auslenkung entspricht, welche ein Operateur bei fixierten Gelenken gegen die Wirkung der von der Steuerung über die angetriebenen Gelenke erzeugten rückstellenden Kraft für eine deutlich über den physiologischen Tremor hinausgehende Positionsänderung vornimmt, um anzuzeigen, dass er das Instrument oder den Manipulatorarm bedienen möchte. Ist der erste Messwert größer als der zweite - und damit auch als der erste - Schwellwert, so erfolgt eine Freigabe zur Bewegung des Instruments, d. h. es wird eine Steuerung durch den Operateur zugelassen und eine möglicherweise vorher eingestellte Fixierung der Gelenke aufgehoben. Der zweite Schwellwert kann beispielsweise als eine Mindestamplitude einer definierten Aktivierungsbewegung vorgeben sein. Ist nur der erste Schwellwert definiert, erfolgt die Freigabe bereits, wenn der erste Messwert größer als der erste Schwellwert ist, beispielsweise bei ausreichend großen Auslenkungen gegen die rückstellende Kraft, wie oben bereits beschrieben.

Um die Sicherheit weiter zu erhöhen, ist es zweckmäßig, die Freigabe zur Bewegung nur dann zuzulassen, wenn zusätzlich anhand weiterer, vorab definierter Kriterien bestätigt wird, dass der Operateur eine Arbeitsposition eingenommen hat. Ein solches Kriterium kann beispielsweise darin bestehen, dass überprüft wird, ob der Operateur seinen Kopf auch in einer Arbeitsposition hat, d. h. sich im Einblick auf einen entsprechenden Bildschirm, der ein Detail des Operationsbereichs am Patienten zeigt, befindet.

Oft ist es so, dass zur Steuerung des Instruments mehr als ein Freiheitsgrad, der sich mit einem einfachen Gelenk realisieren lässt, notwendig ist. Eine Pinzette oder auch eine Schere kann beispielsweise zum einen geöffnet und geschlossen werden und zum anderen um eine Längsachse gedreht werden. In einer weiteren vorteilhaften Ausgestaltung ist das Handstück daher zusätzlich mit einem zweiten passiven Gelenk ausgebildet, welches mit einem zweiten Lagesensor ausgestattet ist. Dann wird analog zur Behandlung der Positionssignale des ersten Lagesensors aus einer zeitlichen Abfolge von Positionssignalen des zweiten Lagesensors ein zweiter Messwert berechnet und mit dem ersten Schwellwert verglichen. Falls auch ein zweiter Schwellwert - wie oben beschrieben - definiert ist, wird der zweite Messwert auch mit dem zweiten Schwellwert verglichen. Die Kriterien zur Unterbrechung der Steuerung der Bewegung des Instruments sowie zur Freigabe der Bewegung werden analog angewendet, wobei die Steuerung nur dann unterbrochen wird, wenn sowohl der erste als auch der zweite Messwert kleiner als der erste Schwellwert sind. Andererseits reicht es aber aus, wenn entweder der erste Messwert oder der zweite Messwert den ersten Schwellwert - bzw. den zweiten Schwellwert, falls dieser definiert ist - überschreiten, d. h. also größer als dieser sind, um die Unterbrechung der Steuerung aufzuheben. Nur an einem der Gelenke muss also der erste bzw. zweite Schwellwert überschritten werden, um die Steuerung wieder dem Operateur zu übergeben. Dies kann beispielsweise der Fall sein, wenn die Drehlage einer Pinzette stabil bleiben soll, sie jedoch geöffnet oder geschlossen werden soll.

Alternativ oder ergänzend kann vorgesehen sein, dass die Steuerung vor der Freigabe mindestens ein zusätzliches Aktivierungssignal empfangen muss, wobei das Aktivierungssignal unabhängig von einer Bewegung ist. Beispielsweise kann es sich um einen Fußschalter handeln, der getätigt werden muss, oder um einen gesonderten Knopf, welcher gedrückt werden muss. Dies erhöht die Sicherheit bei der Kontrolle des Operationssystems.

Zudem ist es möglich, den ersten und/oder den zweiten Schwellwert für den ersten Lagesensor und den zweiten Lagesensor verschieden vorzugeben, was beispielsweise dann vorteilhaft ist, wenn das erste passive Gelenke und das zweite passive Gelenke jeweils verschiedene Typen von Gelenken realisieren. Beispielsweise kann es sich bei dem ersten passiven Gelenk um ein Drehgelenk handeln, mit welchem ein Instrument um eine Instrumentenlängsachse gedreht wird, und beim zweiten passiven Gelenk um ein Schubgelenk, oder um ein Drehgelenk, mit dem das Instrument - beispielsweise eine Pinzette - geöffnet oder geschlossen wird.

Ein robotisches Operationssystem, welches die oben genannte Aufgabe löst, umfasst mindestens einen Manipulatorarm und ein vom Manipulatorarm gehaltenes Instrument. Das robotische Operationssystem umfasst außerdem eine Eingabeeinheit zu Bedienung des mindestens einen Manipulatorarms, wobei die Eingabeeinheit eine mehrgelenkige, kinematische Kette mit aktiven und/oder passiven Gelenken aufweist sowie außerdem ein offenes Ende. Das robotische Operationssystem umfasst darüber hinaus eine Steuereinheit, welche Bewegungen der Eingabeeinheit in Bewegungen des Manipulatorarms und des Instruments umsetzt. Das offene Ende der Eingabeeinheit ist dabei als Handstück zur Steuerung von Bewegungen des Instruments durch einen Operateur ausgebildet. Es umfasst mindestens ein erstes, in der Regel aber mindestens ein erstes und ein zweites passives Gelenk, da nur wenige Instrumente mit einem einzigen durch ein Gelenk einstellbaren Freiheitsgrad konzipiert sind. Das erste passive Gelenk ist mit einer ersten Sensoreinheit ausgestattet, welche einen ersten Lagesensor umfasst. Das zweite passive Gelenk ist mit einer zweiten Sensoreinheit ausgestattet, welche einen zweiten Lagesensor umfasst. Die erste Sensoreinheit ist so ausgebildet, dass sie zeitlich aufeinanderfolgend erste Positionssignale des ersten Lagesensors an die Steuereinheit übermittelt. Analog ist die zweite Sensoreinheit so ausgebildet, dass sie zeitlich aufeinanderfolgend zweite Positionssignale des zweiten Lagesensors an die Steuereinheit übermittelt. Die Steuereinheit ist so ausgebildet, dass sie aus der zeitlichen Abfolge der ersten Positionssignale einen ersten Messwert und aus der zeitlichen Abfolge der zweiten Positionssignale einen zweiten Messwert berechnet. Die Steuereinheit ist außerdem so ausgebildet, dass sie die Steuerung der Bewegung des Instruments unterbricht, wenn der erste Messwert und, falls vorhanden, der zweite Messwert kleiner als ein vorgegebener erster Schwellwert sind.

Was den ersten und den zweiten Messwert sowie den ersten Schwellwert betrifft, gelten die gleichen Ausführungen, wie sie bereits für das Verfahren gemacht wurden, analog. Insbesondere ist der erste Schwellwert so vorgegeben, dass ein Unterschreiten des ersten Schwellwerts durch den ersten und zweiten Messwert das Vorliegen eines physiologischen Tremors und damit einer unwillkürlichen Bewegung ausschließt. Auch kann ein zweiter Schwellwert definiert sein, welcher größer als der erste Schwellwert ist und so vorgegeben ist, dass ein Überschreiten des zweiten Schwellwerts durch den ersten oder zweiten Messwert einem Vorliegen einer bewusst vom Operateur initiierten Bewegung entspricht. Erster und zweiter Schwellwert können auch für beide Lagesensoren verschieden sein. Ist kein zweiter Schwellwert vorgegeben, so entspricht bereits ein Überschreiten des ersten Schwellwerts durch den ersten oder zweiten Messwert einem Vorliegen einer bewusst vom Operateur initiierten Bewegung.

Bevorzugt umfasst das robotische Operationssystem außerdem Fixierungsmittel, welche in dem Fall, dass der erste Messwert und der zweite Messwert den ersten Schwellwert unterschreiten, zumindest die aktiven Gelenke in ihren aktuellen Positionen fixieren. Dabei kann es sich beispielsweise um ein spezielles mechanisches Element handeln, was verhindert, dass sich die Glieder an einem Gelenk gegeneinander bewegen. Es kann sich aber auch um ein spezielles, insbesondere softwaretechnisch realisiertes Element der Steuerung im Zusammenspiel mit einer Motorbremse handeln, in welchem Fall die Gelenke von der Steuerung in ihren aktuellen Positionen fixiert werden, vorzugsweise unter Wirkung einer rückstellenden Kraft. Mittels einer optional vorgesehenen Zeitschaltung wird - sofern die Gelenke fixiert sind - nach Ablauf einer vorgegebenen Zeitspanne die Kopplung zwischen einer Kontrolleinheit, in welche die Eingabeeinheit und in der Regel die Steuereinheit integriert sind, und dem mindestens einen Manipulatorarm aufgehoben, d. h. dass Bewegungen der Eingabeeinheit nicht mehr auf den Manipulatorarm übertragen werden.

Es versteht sich, dass die vorstehend genannten und die nachstehend noch zu erläuternden Merkmale nicht nur in den angegebenen Kombinationen, sondern auch in anderen Kombinationen oder in Alleinstellung einsetzbar sind, ohne den Rahmen der vorliegenden Erfindung zu verlassen.

### Kurze Beschreibung der Zeichnungen

Nachfolgend wird die Erfindung anhand von Ausführungsbeispielen unter Bezugnahme auf die beigefügten Zeichnungen, die ebenfalls erfindungswesentliche Merkmale offenbaren, noch näher erläutert. Diese Ausführungsbeispiele dienen lediglich der Veranschaulichung und sind nicht als einschränkend auszulegen. Beispielsweise ist eine Beschreibung eines Ausführungsbeispiels mit einer Vielzahl von Elementen oder Komponenten nicht dahingehend auszulegen, dass alle diese Elemente oder Komponenten zur Implementierung notwendig sind. Vielmehr können andere Ausführungsbeispiele auch alternative Elemente und Komponenten, weniger Elemente oder Komponenten oder zusätzliche Elemente oder Komponenten enthalten. Elemente oder Komponenten verschiedener Ausführungsbespiele können miteinander kombiniert werden, sofern nichts anderes angegeben ist. Modifikationen und Abwandlungen, welche für eines der Ausführungsbeispiele beschrieben werden, können auch auf andere Ausführungsbeispiele anwendbar sein. Zur Vermeidung von Wiederholungen werden gleiche oder einander entsprechende Elemente in verschiedenen Figuren mit gleichen Bezugszeichen bezeichnet und nicht mehrmals erläutert. Es zeigen:
Fig. 1 eine Übersichtszeichnung eines robotischen Operationssystems,
Fig. 2 eine detailliertere Darstellung einer Kontrolleinheit des Operationssystems,
Fig. 3 eine Eingabeeinheit als Teil der Kontrolleinheit im Detail,
Fig. 4 eine Ausgestaltung eines Handstücks, und
Fig. 5 einen typischen Verfahrensablauf bei der Steuerung des Operationssystems.

### Ausführliche Beschreibung der Zeichnungen

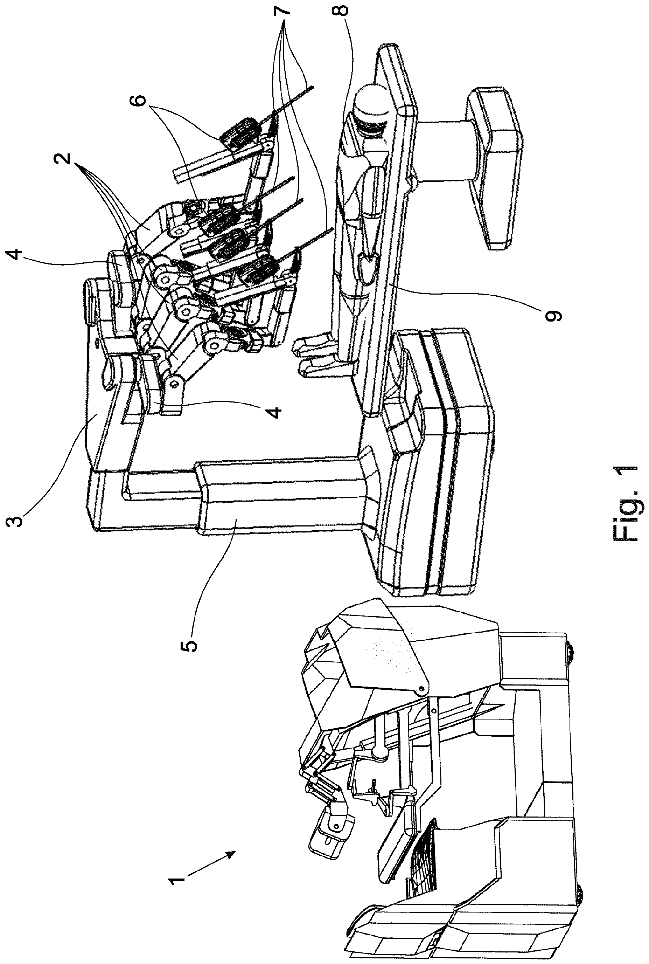

**Fig. 1** zeigt zunächst eine Gesamtansicht eines robotischen Operationssystems. Auf der linken Seite ist eine Kontrolleinheit 1 gezeigt, von welcher aus ein Operateur einen oder mehrere Manipulatorarme 2 bedient, welche an einer Traverse 3 über Koppelvorrichtungen 4 befestigt sind. Die Traverse 3 ist an einer Tragsäule 5 befestigt, welche in der Höhe verstellbar ist. Die Manipulatorarme 2 sind als kinematische Ketten ausgebildet und mit ihrem einen Ende an der Traverse 3 befestigt. An den anderen, offenen Enden sind Instrumentenhalter 6, welche Instrumente 7 aufnehmen, beweglich angekoppelt. Es handelt sich hierbei nur um eine Prinzipzeichnung, in der Realität unterscheiden sich die Instrumente 7 - beispielsweise Scheren, Pinzetten, Haken oder Endoskope - voneinander. Der Operateur bedient von der Kontrolleinheit 1 den unteren Teil der Manipulatorarme 2 mit den Instrumenten 7 und operiert einen Patienten 8, der auf einer Patientenliege 9 liegt.

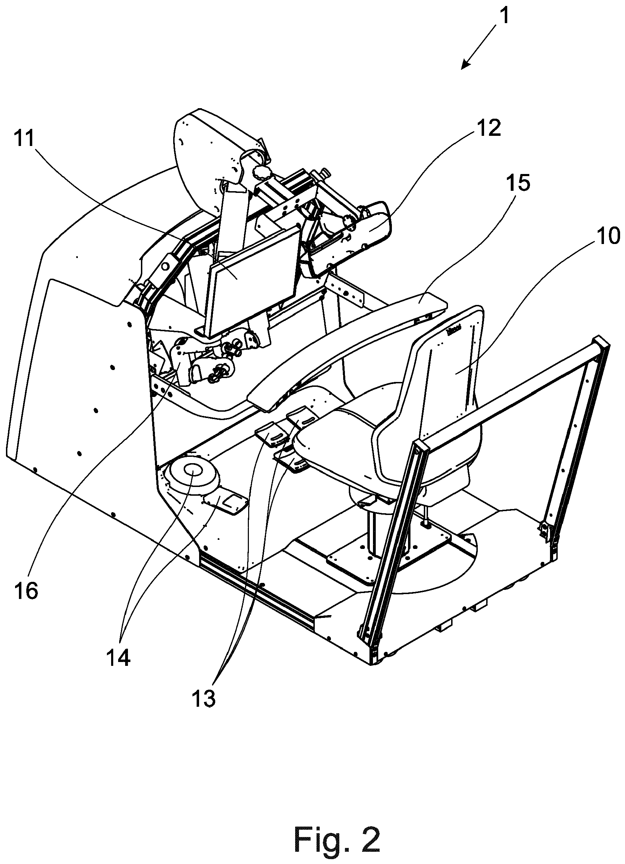

Die Kontrolleinheit 1 ist beweglich und wird im Folgenden anhand von **Fig. 2** näher erläutert. In ihr ist eine von außen nicht sichtbare und daher in Fig. 2 nicht gezeigte Steuereinheit verbaut. Der Operateur nimmt auf einem verstellbaren Stuhl 10 Platz. Einen Überblick über das Operationsgebiet erhält er über einen Übersichtsbildschirm 11. Rechts daneben befindet sich ein Operateur-Bildschirm 12, auf welchem der Operateur einen Ausschnitt des Operationsgebiets im Detail sieht, in welchem aktuell operiert wird. Der Operateur-Bildschirm 12 umfasst außerdem Mittel zur Erkennung, ob der Operateur in Position ist und Einblick auf den Operateur-Bildschirm 12 nimmt. Die Mittel zur Erkennung können beispielsweise eine Eye-Tracking-Vorrichtung umfassen oder andere Sensoren, die erkennen, ob ein Kopf im Einblick ist, beispielsweise optische Annäherungssensoren mit Distanzmessung, oder eine einfache Lichtschranke. Zur Bedienung der Manipulatorarme 2 stehen außerdem verschiedene Fußschalter 13, 14 zur Verfügung, die auch einen Wechsel der Bedienung zwischen zwei Manipulatorarmen 2 initiieren können. Während der Operation kann der Operateur seine Ellbogen oder Unterarme auf einer Leiste 15 abstützen.

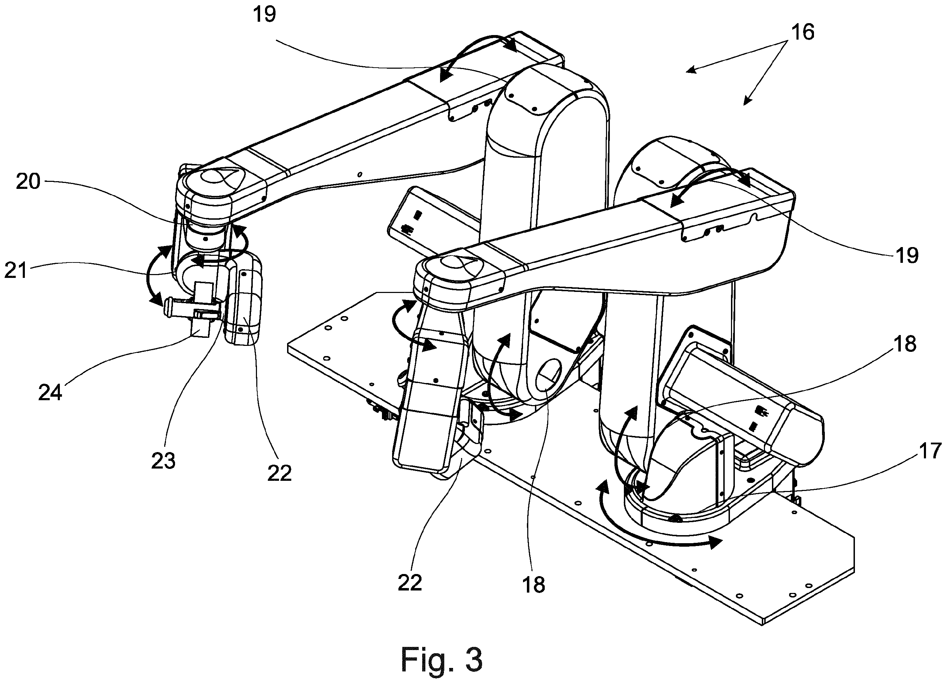

Die Steuerung bzw. Bedienung der unteren Teile der Manipulatorarme 2 mit den Instrumenten 7 erfolgt über eine Eingabeeinheit 16, welche anhand von **Fig. 3** näher erläutert wird. Diese zeigt zwei Eingabeeinheiten 16 zur Bedienung von zwei Manipulatorarmen 2. Da die Manipulatorarme 2 als kinematische Kette ausgestaltet sind, ist es zweckmäßig, wenn auch die Eingabeeinheiten 16 zur Bedienung eines Manipulatorarms 2 jeweils eine mehrgelenkige kinematische Kette mit aktiven und/oder passiven Gelenken aufweisen sowie ein offenes Ende. Die Bewegungen der Eingabeeinheiten 16 werden von der Steuereinheit in Bewegungen des Manipulatorarms 2 und des Instruments 7 umgesetzt. Die hier gezeigten Eingabeeinheiten 16 sind als kinematische Kette mit insgesamt jeweils fünf Gelenken 17, 18, 19, 20, 21 ausgebildet, deren Positionen in **Fig. 3** nur angedeutet sind. Es handelt sich jeweils um Rotationsgelenke mit einem Freiheitsgrad, die Rotationsrichtung wird durch die Doppelpfeile bei den entsprechenden Gelenken angedeutet. Die Gelenke 17-21 sind im gezeigten Beispiel aktive Gelenke, verfügen also über eine Motorunterstützung. Die drei Gelenke 17-19 steuern, wenn sie mit einem Manipulatorarm 2 gekoppelt sind, die Lage des Instruments 7 im Raum.

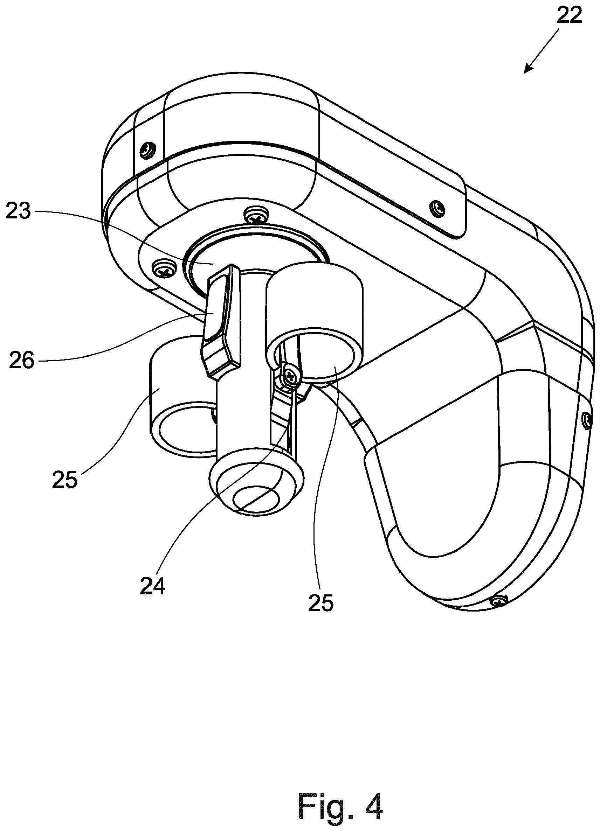

Das offene Ende der Eingabeeinheit 16 ist jeweils als Handstück 22 zur Steuerung von Bewegungen des Instruments 7 durch den Operateur ausgebildet. Das hier beispielhaft ausgewählte Handstück 22 ist in **Fig. 4** im Detail dargestellt. Es ist für die Verwendung mit einem Instrument 7, welches über ein Maulteil verfügt, ausgelegt. Solche Instrumente sind beispielsweise Pinzetten oder Scheren, im Folgenden wird die Funktion nur zur Veranschaulichung anhand einer Pinzette erläutert. Es umfasst ein erstes passives Gelenk 23 und ein zweites passives Gelenk 24. Mit den Gelenken 20-24 wird die Bewegung der Instrumentenspitze gesteuert, wenn die Lage des Instrumentes im Raum eingestellt und fixiert wurde. Mithilfe des ersten passiven Gelenks 23 wird die Drehung des Instruments 7 um seine Längsachse realisiert. Das erste passive Gelenke 23 ist mit einer - hier nicht gezeigten - ersten Sensoreinheit im Innern des Handstücks 22 ausgestattet, welche einen ersten Lagesensor umfasst. Der erste Lagesensor erfasst dabei die Drehlage des ersten passiven Gelenks 23 in einem Bereich zwischen 0° und 360°. Das zweite passive Gelenk 24 ist mit einer - hier ebenfalls nicht gezeigten - zweiten Sensoreinheit im Innern des Handstücks 22 ausgestattet, welche einen zweiten Lagesensor umfasst. Für beide passiven Gelenke 23 und 24 werden die Positionen mittels eines Absolut-Encoders absolut bestimmt, so dass die Winkelauslenkungen ohne Vergleich mit einem Referenzwert ausgegeben werden können. Das zweite passive Gelenk 24 verfügt über zwei Fingerschlaufen 25, in welche der Operateur beispielsweise Daumen und Zeigefinger oder Mittelfinger stecken kann, um die Pinzette zu öffnen und zu schließen. Ein geeigneter Mechanismus ist beispielsweise in der DE 10 2017 103 199 A1 beschrieben. Der Öffnungswinkel - entsprechend einer Auslenkung der Fingerschlaufen aus einer Ruheposition - wird dabei über den zweiten Lagesensor, der in die Bewegungsmechanik integriert sein kann, ermittelt. An dem Handstück 22 befindet sich außerdem noch eine Fingerkopplung 26, mit welcher - neben weiteren Funktionen - eine Aktivierungsbewegung durchgeführt werden kann, die der Steuereinheit signalisiert, dass ein Operateur die Kontrolle über die jeweilige Eingabeeinheit 16 übernimmt. Die Steuereinheit gibt die Kontrolle dann frei, sofern der Operateur seinen Kopf im Einblick auf den Operateur-Bildschirm 12 hat. Mit dem Handstück 22 lassen sich selbstverständlich auch andere Instrumente mit nur einem Freiheitsgrad, beispielsweise Endoskope, steuern. Hier wird bei angewinkelter Abstrahlfläche nur die Möglichkeit der Drehung um die Längsachse mittels des ersten passiven Gelenks 23 benötigt; eine Bewegung des zweiten passiven Gelenks 24 hat dann keine das Instrument 7 steuernde Wirkung.

Die erste Sensoreinheit übermittelt nun zeitlich aufeinanderfolgend erste Positionssignale des ersten Lagesensors an die Steuereinheit, entsprechend übermittelt die zweite Sensoreinheit zeitlich aufeinanderfolgend zweite Positionssignale des zweiten Lagesensors an die Steuereinheit. Die Steuereinheit ermittelt aus der zeitlichen Abfolge der ersten Positionssignale einen ersten Messwert und aus der zeitlichen Abfolge der zweiten Positionssignale einen zweiten Messwert. Sind der erste Messwert und der zweite Messwert kleiner als ein vorgegebener erster Schwellwert, so unterbricht die Steuereinheit die Steuerung der Bewegungen des Instruments. Der erste Schwellwert ist dabei so vorgegeben, dass ein Unterschreiten des ersten Schwellwerts durch den ersten und den zweiten Messwert das Vorliegen eines physiologischen Tremors und damit einer unwillkürlichen Bewegung ausschließt. Dies setzt voraus, dass die Empfindlichkeit des ersten Lagesensors und des zweiten Lagesensors hoch genug ist, um einen physiologischen Tremor bzw. die damit verbundenen Amplituden der Auslenkung zu detektieren. Eine Auflösung des ersten Lagesensors von 16 Bit - entsprechend einer Winkelauflösung von 0,005° pro Inkrement - hat sich beispielsweise als ausreichende Auflösung erwiesen. Der erste Schwellwert kann dann beispielsweise bei 0,005° als unterer Grenze festgelegt werden, er kann aber auch einige wenige Inkremente betragen. Der erste Schwellwert kann sich auch für den ersten Lagesensor und den zweiten Lagesensor unterscheiden, d. h. jedem der Lagesensoren kann ein eigener erster Schwellwert zugeordnet werden. Dies ist insbesondere dann vorteilhaft, wenn die Gelenke deutlich voneinander verschieden sind.

Für die Berechnung des ersten Messwerts und des zweiten Messwerts gibt es verschiedene Möglichkeiten. Die einfachste Möglichkeit besteht darin, zwei aufeinanderfolgende Positionssignale voneinander zu subtrahieren und zu überprüfen, ob die Differenz größer oder kleiner als der erste Schwellwert ist. Die Genauigkeit lässt sich jedoch erhöhen, wenn man eine längere zeitliche Folge von Positionssignalen analysiert. Beispielsweise können fortlaufend die Standardabweichungen der Positionen des ersten passiven Gelenks und des zweiten passiven Gelenks bestimmt werden und diese als erster bzw. zweiter Messwert festgelegt werden. Besonders vorteilhaft ist es, dabei länger zurückliegende Positionssignale schwächer zu berücksichtigen und mit einem Dämpfungsfaktor zu belegen.

Zusätzlich kann ein zweiter Schwellwert vorgegeben werden, mit welchem der erste und der zweite Messwert ebenfalls verglichen werden. Der zweite Schwellwert wird dabei so vorgegeben, dass ein Überschreiten des zweiten Schwellwerts durch den ersten Messwert und/oder den zweiten Messwert dem Vorliegen einer bewusst vom Operateur initiierten Bewegung entspricht. Der zweite Schwellwert ist daher deutlich größer als der erste Schwellwert. Überschreitet einer der beiden Messwerte den zweiten Schwellwert, so kann dies von der Steuereinheit auch als Aktivierung der Eingabeeinheit zur Übernahme der Kontrolle durch den Operateur interpretiert werden, wenn zwar erkannt wurde, dass der Operateur das Handstück losgelassen hat, aber eine Zeitspanne bis zur Unterbrechung der Steuerung und damit der Kopplung zwischen Eingabeeinheit und Manipulatorarm noch nicht abgelaufen ist. Ein zusätzliches Aktivierungssignal ist in diesem Fall nicht nötig.

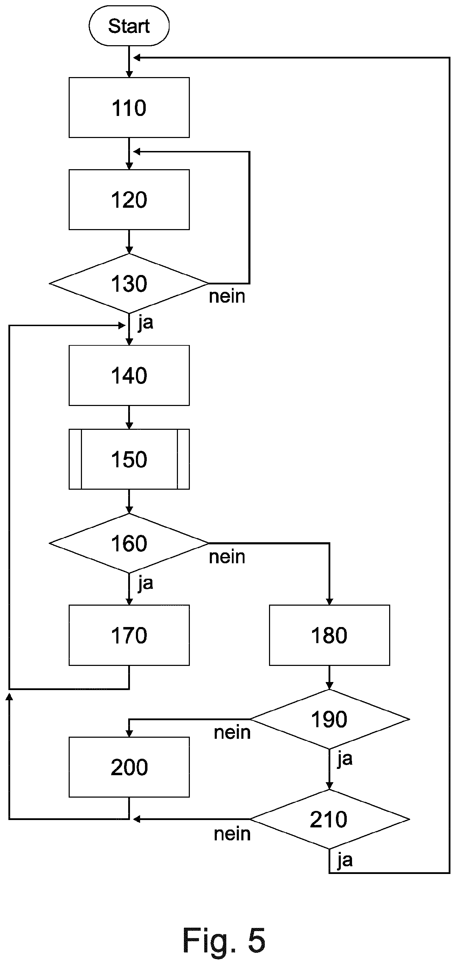

Der Ablauf eines Verfahrens zur Bedienung eines robotischen Operationssystems, bei dem erkannt wird, ob ein Operateur die Eingabeeinheit steuert oder nicht, wird im Folgenden anhand von **Fig. 5** erläutert. Beim Start des Systems wird zunächst in einem Schritt 110 die Möglichkeit der Steuerung der Eingabeeinheit 16 durch den Operateur von der Steuereinheit blockiert. In einer zeitlichen Schleife im Schritt 120 wartet die Steuereinheit darauf, dass der Operateur ein Aktivierungssignal auslöst. Dieses Aktivierungssignal kann beispielsweise ausgelöst werden, indem der Operateur die Fingerkopplung 26 entlang der Achse vom Handstück 22 wegzieht. Alternativ lässt sich ein Aktivierungssignal auch durch eine größere Auslenkung des zweiten passiven Gelenks 24 mittels der in die Fingerschlaufen 25 gesteckten Autopodien erreichen, welche einen Messwert liefert, der den zweiten Schwellwert überschreitet. Für diese Aktivierungsbewegung gibt es zwei Möglichkeiten. Ist die Instrumentenspitze geschlossen, so werden zur Aktivierung die Fingerschlaufen 25 voneinander wegbewegt und dann wieder aufeinander zu bewegt, was einem Öffnen entspricht. Ist die Instrumentenspitze hingegen geöffnet, so werden zur Aktivierung die Fingerschlaufen 25 aufeinander zu bewegt, was einem Schließen entspricht, und dann wieder voneinander wegbewegt. Damit der Operateur die Kontrolle über die Eingabeeinheit 16 übernehmen kann, muss er sich außerdem noch im Einblick auf den Operateur-Bildschirm 12 befinden. In einem Schritt 130 überprüft die Steuereinheit, ob alle Bedingungen für die Übernahme der Kontrolle durch den Operateur erfüllt sind. Ist dies nicht der Fall, so wird die Schleife in Schritt 120 erneut durchlaufen.

Wird jedoch die Aktivierung bestätigt, so erfolgt die Übernahme der Kontrolle der Eingabeeinheit 16 durch den Operateur. In der Folge übermitteln die erste und zweite Sensoreinheit in Schritt 140 kontinuierlich Positionssignale des ersten und zweiten Lagesensors an die Steuereinheit. In Schritt 150 berechnet die Steuereinheit aus der zeitlichen Abfolge der Positionssignale des ersten Lagesensors einen ersten Messwert und aus der zeitlichen Abfolge der Positionssignale des zweiten Lagesensors einen zweiten Messwert und vergleicht diese Messwerte mit dem ersten Schwellwert. Dieser ist so vorgegeben, dass ein Unterschreiten des ersten Schwellwerts sowohl durch den ersten Messwert als auch durch den zweiten Messwert eine unwillkürliche Bewegung der Elemente des Handstücks 22, verursacht durch einen physiologischen Tremor des Operateurs, ausschließt.

In Schritt 160 wird abgefragt, ob der Operateur die Eingabeeinheit 16 weiterhin kontrolliert. Dies ist der Fall, wenn mindestens einer der beiden Messwerte größer als der erste Schwellwert ist. In diesem Fall wird Schritt 170 ausgeführt und der Operateur bleibt in der Kontrolle der Eingabeeinheit, d. h. es erfolgt keine Fixierung der aktiven und/oder passiven Gelenke und die Daten der beiden Lagesensoren werden weiter ausgelesen. Der Operateur kontrolliert die Eingabeeinheit 16 jedoch nicht, wenn beide Messwerte kleiner als der erste Schwellwert sind. In diesem Fall wird Schritt 180 ausgeführt und die Steuereinheit übernimmt die Kontrolle zumindest über die aktiven Gelenke der Eingabeeinheit 16, und die aktiven Gelenke - hier die Gelenke 17-22 - werden motorisch in ihren aktuellen Positionen fixiert, wobei die Fixierung unter Wirkung einer rückstellenden Kraft erfolgt; dies wird durch die Steuereinheit und die motorischen Antriebe der Gelenke realisiert. Die Gelenke bzw. die Glieder, welche die Gelenke verbinden, verhalten sich in dieser sogenannten Impedanzregelung also wie Körper, welche durch Federn auf ihren Positionen gehalten werden, so dass minimale Positionsänderungen möglich sind, auch um eine erneute Aktivierung zu ermöglichen.

In einem Schritt 190 erfolgt dann die Abfrage, ob bereits eine Zeitschaltuhr gestartet wurde. Ist dies nicht der Fall, so wird in einem Schritt 200 die Zeitschaltuhr gestartet und es werden weiterhin die Positionssignale der beiden Lagesensoren ausgewertet. Sollte sich dabei ergeben, dass mindestens einer der beiden Messwerte größer als der erste Schwellwert ist, was bedeutet, dass der Operateur das Handstück 22 noch nicht losgelassen hat, so wird die Zeitschaltuhr gestoppt und dem Operateur wieder die volle Kontrolle über die Eingabeeinheit 16 gewährt. Aufgrund der Wirkung der rückstellenden Kraft verhält sich die Eingabeeinheit 16 in diesem Fall so, dass der Operateur nicht beeinträchtigt wird.

Für den Fall, dass die Zeitschaltuhr bereits gestartet wurde, erfolgt nach Schritt 190 in einem Schritt 210 dann die Abfrage, ob eine vorgegebene Zeitspanne - in der Regel beträgt diese Zeitspanne zwischen drei Sekunden und fünf Sekunden, es können aber auch kleinere oder größere Werte vorgegeben werden - abgelaufen ist. In diesem Fall wird zusätzlich zu der Fixierung der Gelenke der Eingabeeinheit 16 auch die Steuerung zwischen der Eingabeeinheit 16 und den Manipulatorarmen 2 unterbrochen und es wird mit Schritt 110 fortgefahren. Möchte der Operateur weiterarbeiten, so muss er daher erneut eine Aktivierungsbewegung durchführen.

Mit dem vorangehend beschriebenen Verfahren und einem dieses Verfahren umsetzenden robotischen Operationssystem kann im Wesentlichen ohne zusätzliche technische Komponenten und mit hoher Genauigkeit eine Kontrolle realisiert werden, die feststellt, ob ein Operateur zur Bedienung der Eingabeeinheit des Operationssystems bereit ist oder nicht. Dazu werden Daten - die Positionssignale von Lagesensoren - verwendet, welche sowieso bei der Bedienung des robotischen Operationssystems und insbesondere des Instruments anfallen; diese Daten werden einer zusätzlichen Auswertung, die rechnerisch erfolgen kann, unterzogen.

### Bezugszeichenliste

- 1: Kontrolleinheit
- 2: Manipulatorarm
- 3: Traverse
- 4: Koppelvorrichtung
- 5: Tragsäule
- 6: Instrumentenhalter
- 7: Instrument
- 8: Patient
- 9: Patientenliege
- 10: verstellbaren Stuhl
- 11: Übersichtsbildschirm
- 12: Operateur-Bildschirm
- 13: Fußschalter
- 14: Fußschalter
- 15: Leiste
- 16: Eingabeeinheit
- 17: Gelenk
- 18: Gelenk
- 19: Gelenk
- 20: Gelenk
- 21: Gelenk
- 22: Handstück
- 23: erstes passives Gelenk
- 24: zweites passives Gelenk
- 25: Fingerschlaufe
- 26: Fingerkopplung

## Patentansprüche

1. Verfahren zur Steuerung mindestens eines Manipulatorarms (2) eines robotischen Operationssystems und eines von dem mindestens einen Manipulatorarm (2) gehaltenen Instruments (7) mittels einer Eingabeeinheit (16), welche eine mehrgelenkige, kinematische Kette mit aktiven und/oder passiven Gelenken (17, 18, 19, 20, 21) sowie ein offenes Ende aufweist, wobei das offene Ende als Handstück (22) mit mindestens einem ersten passiven Gelenk (23) ausgebildet ist, über dessen Bewegung ein Operateur Bewegungen des Instruments (7) steuert, wobei das erste passive Gelenk (23) mit einem ersten Lagesensor ausgestaltet ist und Positionssignale des ersten Lagesensors zur Steuerung der Bewegung des Instruments (7) verwendet werden,
- wobei aus einer zeitlichen Abfolge der Positionssignale des ersten Lagesensors ein erster Messwert berechnet und mit mindestens einem vorgegebenen ersten Schwellwert verglichen wird, und
- wobei die Steuerung der Bewegung des Instruments (7) unterbrochen wird, falls der erste Messwert kleiner als der erste Schwellwert ist.

2. Verfahren nach Anspruch 1, bei dem der erste Schwellwert so vorgegeben wird, dass ein Unterschreiten des ersten Schwellwerts durch den ersten Messwert das Vorliegen eines physiologischen Tremors und damit einer unwillkürlichen Bewegung ausschließt.

3. Verfahren nach einem der Ansprüche 1 oder 2, bei dem bei der Unterbrechung der Steuerung der Bewegung des Instruments (7) zumindest die aktiven Gelenke in ihren aktuellen Positionen fixiert werden.

4. Verfahren nach Anspruch 3, **dadurch gekennzeichnet, dass** zumindest die aktiven Gelenke unter Wirkung einer rückstellenden Kraft fixiert werden.

5. Verfahren nach einem der Ansprüche 1 bis 4, **dadurch gekennzeichnet, dass** die Steuerung erst nach Ablauf einer vorgegebenen Zeitspanne unterbrochen wird.

6. Verfahren nach einem der Ansprüche 1 bis 5, bei dem die Unterbrechung der Steuerung aufgehoben wird und eine Freigabe der Bewegung des Instruments (7) erfolgt, wenn der erste Messwert größer als der erste Schwellwert ist und/oder wenn von der Steuereinheit ein von der Bewegung unabhängiges Aktivierungssignal empfangen wird

7. Verfahren nach einem der Ansprüche 1 bis 6, bei dem der erste Messwert zusätzlich mit einem zweiten Schwellwert verglichen wird und der zweite Schwellwert so vorgegeben wird, dass ein Überschreiten des zweiten Schwellwerts durch den ersten Messwert einem Vorliegen einer bewusst vom Operateur initiierten Bewegung entspricht, wobei der zweite Schwellwert größer als der erste Schwellwert ist.

8. Verfahren nach Anspruch 7, bei dem die Unterbrechung der Steuerung aufgehoben wird und eine Freigabe zur Bewegung des Instruments (7) erfolgt, wenn der erste Messwert größer als der zweite Schwellwert ist.

9. Verfahren nach Anspruch 6 oder 8, bei dem die Freigabe nur erfolgt, wenn anhand zusätzlicher, vorab definierter Kriterien bestätigt wird, dass der Operateur eine Arbeitsposition eingenommen hat.

10. Verfahren nach einem der Ansprüche 1 bis 9, bei dem das Handstück (22) zusätzlich mit einem zweiten passiven Gelenk (24) ausgebildet ist, welches mit einem zweiten Lagesensor ausgestattet ist, wobei aus einer zeitlichen Abfolge von Positionssignalen des zweiten Lagesensors ein zweiter Messwert berechnet und mit dem ersten Schwellwert, und falls definiert, mit dem zweiten Schwellwert verglichen wird,
- wobei die Steuerung der Bewegung des Instruments (7) unterbrochen wird, falls der erste und der zweite Messwert kleiner als der erste Schwellwert sind, und
- wobei die Unterbrechung der Steuerung aufgehoben wird, wenn entweder der erste Messwert oder der zweite Messwert den ersten Schwellwert, und falls definiert, den zweiten Schwellwert überschreiten und/oder wenn von der Steuereinheit ein von einer Bewegung unabhängiges Aktivierungssignal empfangen wird.

11. Robotisches Operationssystem, umfassend mindestens einen Manipulatorarm (2) und ein vom Manipulatorarm (2) gehaltenes Instrument (7), eine Eingabeeinheit (16) zur Bedienung des mindestens eines Manipulatorarms (2), wobei die Eingabeeinheit (16) eine mehrgelenkige, kinematische Kette mit aktiven und/oder passiven Gelenken (17, 18, 19, 20, 21) sowie ein offenes Ende aufweist, sowie eine Steuereinheit, welche Bewegungen der Eingabeeinheit (16) in Bewegungen des Manipulatorarms (2) und des Instruments (7) umsetzt,
- wobei das offene Ende der Eingabeeinheit (16) als Handstück (22) zur Steuerung von Bewegungen des Instruments (7) durch einen Operateur ausgebildet ist,
- wobei das Handstück (22) mindestens ein erstes passives Gelenk (23) und ein zweites passives Gelenk (24) umfasst, und das erste passive Gelenk (23) mit einer ersten Sensoreinheit umfassend einen ersten Lagesensor und das zweite passive Gelenk (24) mit einer zweiten Sensoreinheit umfassend einen zweiten Lagesensor ausgestattet ist,
- wobei die erste Sensoreinheit zeitlich aufeinanderfolgend erste Positionssignale des ersten Lagesensors an die Steuereinheit übermittelnd ausgebildet ist und die zweite Sensoreinheit zeitlich aufeinanderfolgend zweite Positionssignale des zweiten Lagesensors an die Steuereinheit übermittelnd ausgebildet ist,
- wobei die Steuereinheit aus der zeitlichen Abfolge der ersten Positionssignale einen ersten Messwert und aus der zeitlichen Abfolge der zweiten Positionssignale einen zweiten Messwert berechnend ausgebildet ist, und
- wobei die Steuereinheit die Steuerung der Bewegungen des Instruments (7) unterbrechend ausgebildet ist, wenn der erste Messwert und der zweite Messwert kleiner als ein vorgegebener erster Schwellwert sind.

12. Robotisches Operationssystem nach Anspruch 11, bei dem der erste Schwellwert so vorgegeben ist, dass ein Unterschreiten des ersten Schwellwerts durch den ersten und zweiten Messwert das Vorliegen eines physiologischen Tremors und damit einer unwillkürlichen Bewegung ausschließt.

13. Robotisches Operationssystem nach Anspruch 11 oder 12, umfassend Fixierungsmittel, welche in dem Fall, dass der erste und der zweite Messwert den ersten Schwellwert unterschreiten, zumindest die aktiven Gelenke in ihren aktuellen Positionen fixieren.

14. Robotisches Operationssystem nach Anspruch 13, umfassend eine Zeitschaltung, welche bei Fixierung der aktiven Gelenke nach Ablauf einer vorgegebenen Zeitspanne eine Kopplung zwischen der Eingabeeinheit (16) und dem mindestens einen Manipulatorarm (2) aufhebt.

15. Robotisches Operationssystem nach einem der Ansprüche 11 bis 14, bei dem ein zweiter Schwellwert definiert ist, welcher größer als der erste Schwellwert ist und so vorgegeben ist, dass ein Überschreiten des zweiten Schwellwerts durch den ersten oder zweiten Messwert einem Vorliegen einer bewusst vom Operateur initiierten Bewegung entspricht.
